# EUROPEAN PATENT APPLICATION

(11) **EP 3 590 595 A1**
(43) Date of publication of application: **08.01.2020**
(21) Application number: 18761419.3
(22) Date of filing: 25.01.2018
(51) Int. Cl.: B01J 23/755, C07C 29/17, C07C 29/141, C07C 31/20

(54) **NI-COATED AL203@SI02 CATALYST, PREPARATION METHOD THEREOF AND APPLICATION THEREOF**

(30) Priority: 28.02.2017 CN 201710109983
(71) Applicant: Shanxi University, Taiyuan, Shanxi 030006 (CN)
(72) Inventor: ZHAO, Yongxiang, Taiyuan Shanxi 030006 (CN); LI, Haitao, Taiyuan Shanxi 030006 (CN); SUN, Zijin, Taiyuan Shanxi 030006 (CN); LIU, Shaobo, Taiyuan Shanxi 030006 (CN)
(74) Representative: SSM Sandmair
(86) International application number: PCT/CN2018/074142
(87) International publication number: WO 2018/157684

(57) **Abstract**

A Ni-Al₂O₃@SiO₂ catalyst with coated structure is provided. The catalyst has a mass ratio of respective compositions Al₂O₃:SiO₂:Ni=100: 0.1 ∼ 3: 4 ∼ 26, a specific surface area of 98 m²/g to 245 m²/g, and a pore volume of 0.25 cm³/g to 1.1 cm³/g; Ni particles are distributed on a surface of an Al₂O₃ carrier in an amorphous or highly dispersed state and have a grain size less than or equal to 8 nm, and a SiO₂ layer is filled among the Ni particles. The invention has advantages of easy to prepare and good selectivity.

## Description

### BACKGROUND OF THE INVENTION

### 1. Field of the Invention

The invention relates to a Ni-Al₂O₃@SiO₂ catalyst with coated structure, a preparation method thereof and an application thereof.

### 2. Related Art

Superficially, hydrogenation of 1,4-butynediol to prepare butane-1,4-diol using a Reppe process is a process of hydrogenation of C≡C and C=C bonds to prepare a saturated diol:

HO-CH₂-C≡C-CH₂-OH+H₂→HO-CH₂-CH=CH-CH₂-OH+154.8 KJ/mol (1)

| | |
|---|---|
| 1,4-butynediol | 1,4-butenediol |

HO-CH₂-CH=CH-CH₂-OH+H₂→HO-CH₂-CH₂-CH₂-CH₂-OH+96.3KJ/mol (2)

| | |
|---|---|
| 1,4-butenediol | butane-1,4-diol |

In actual reaction process, it is a complex system containing a plurality of compositions while accompanying with several side reactions. In the system, the product 1,4-butenediol after half hydrogenation may be isomerized to 4-hydroxybutyraldehyde.

HO-CH₂-CH=CH-CH₂-OH → HO-CH₂-CH₂-CH=CH-OH ↔ HO-CH₂-CH₂-CH₂-CHO (3)

As 4-hydroxybutyraldehyde is unstable, it may condense to produce a corresponding hemiacetal:

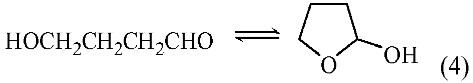

Hemiacetal reacts with butane-1,4-diol to produce acetal, 2-(4'-hydroxybutoxy)-tetrahydrofuran:

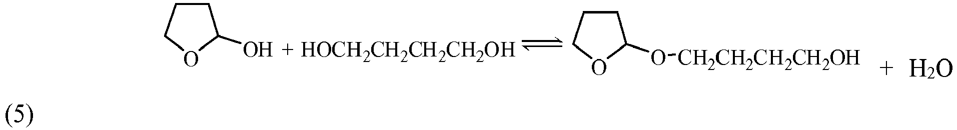

In the above reaction, 1,4-butynediol, 1,4-butenediol, and 4-hydroxybutyraldehyde may be converted to butane-1,4-diol through a simple catalytic hydrogenation process. However, cyclic acetal, 2-(4'-hydroxybutoxy)-tetrahydrofuran cannot be removed by direct hydrogenation, and because it forms an azeotrope with butane-1,4-diol, it is difficult to separate it using conventional distillation method. Remaining of acetal, 2-(4'-hydroxybutoxy)-tetrahydrofuran seriously affects purity and chromaticity of butane-1,4-diol products, and directly affects quality of butane-1,4-diol and its application in the downstream field.

In view of this, US6137016 describes a process for purification of butane-1,4-diol containing a minor amount of cyclic acetal, 2-(4'-hydroxybutoxy)-tetrahydrofuran, wherein a minor amount of water is added into butane-1,4-diol finished product, then hydrogenation reaction is conducted in the presence of a hydrogenation catalyst, and butane-1,4-diol product with a low content of 2-(4'-hydroxybutoxy)-tetrahydrofuran is obtained by further rectification. The method needs to conduct further hydrogenation conversion and product separation to butane-1,4-diol finished product, which increases input of additional equipment and consumption of energy source. Moreover, since the hydrogenation catalyst used therein is a general-purpose hydrogenation catalyst, the hydrogenation effect is not ideal. CN 102145286 B discloses a Ni-SiO₂/Al₂O₃ catalyst and a preparation method thereof. During preparation of the catalyst, silicon additive is firstly loaded onto a surface of an Al₂O₃ carrier for modification, and then active components nickel are loaded. Since there is a weak interaction between SiO₂ and the active components nickel, nickel is loaded using impregnation and deposition methods, so operations are complicated.

### SUMMARY OF THE INVENTION

An object of the invention is to provide a Ni-Al₂O₃@SiO₂ catalyst with coated structure which is easy to prepare and has good selectivity, a preparation method thereof and an application thereof.

In order to achieve the above object, the invention provides a Ni-Al₂O₃@SiO₂ catalyst with coated structure, characterized in that Ni particles are distributed on a surface of an Al₂O₃ carrier in an amorphous or highly dispersed state and have a grain size less than or equal to 8 nm, and a SiO₂ layer is filled among the Ni particles.

Regarding the Ni-Al₂O₃@SiO₂ catalyst with coated structure according to the invention, the catalyst has a mass ratio of respective compositions Al₂O₃:SiO₂:Ni=100: 0.1 ∼ 3: 4 ∼ 26, a specific surface area of 98 m²/g to 245 m²/g, and a pore volume of 0.25 cm³/g to 1.1 cm³/g.

Regarding the Ni-Al₂O₃@SiO₂ catalyst with coated structure according to the invention, the Al₂O₃ carrier has a specific surface area of 110 m²/g to 260 m²/g, and a pore volume of 0.5 cm³/g to 1.3 cm³/g.

In order to achieve the above object, the invention further provides a preparation method of the Ni-Al₂O₃@SiO₂ catalyst with coated structure, comprising steps of:
impregnation step: loading the active components Ni onto the Al₂O₃ carrier using an impregnation method, Ni being distributed in tetrahedral and octahedral holes on the Al₂O₃ surface, and growing into microcrystalline particles by using the tetrahedral and octahedral holes as nuclei;
grafting step: loading the SiO₂ layer in a grafting manner onto a surface of a Ni/Al₂O₃ catalyst obtained in the impregnation step, SiO₂ reacting with Al₂O₃ exposed in gaps of the Ni particles to produce corresponding Al-O-Si bonds; and
washing step: removing weak adsorption compositions remaining on the surface of the catalyst during grafting process by washing the sample after completion of grafting reaction.

In order to achieve the above object, the invention further provides another preparation method of the Ni-Al₂O₃@SiO₂ catalyst with coated structure, comprising the steps of:
(1) cooling the Al₂O₃ carrier to a room temperature for use after treatment at 100 °C to 150 °C;
(2) preparing a nickel salt solution having a nickel content of 0.05 g/mL to 0.2 g/mL;
(3) impregnating the nickel salt solution prepared by step (2) into the carrier of step (1) according to a ratio of 80 mL to 130 mL of nickel salt solution for 100g of the Al₂O₃ carrier, standing, drying and calcining to obtain a NiO/Al₂O₃ precursor;
(4) preparing an ethanol-water solution of silicon precursor with a concentration of 0.0001 g/mL to 0.0015 g/mL by calculating SiO₂, and adjusting a pH value of the ethanol-water solution to 8.0 to 8.5 by ammonia water;
(5) suspending the NiO/Al₂O₃ precursor obtained by step (3) in the solution prepared by step (4) according to an amount of 1000 mL to 2000 mL of ethanol-water solution of silicon precursor for 100g of Al₂O₃ in the precursor, stirring and refluxing at a constant temperature, centrifugal washing respectively with absolute ethyl alcohol, dilute nitric acid and deionized water after filtration, and drying;
(6) calcining the sample obtained by step (5) in an air or nitrogen atmosphere, then conducting reduction under the condition of hydrogen to obtain a catalyst.

In order to achieve the above object, the invention further provides another preparation method of the Ni-Al₂O₃@SiO₂ catalyst with coated structure, comprising the steps of:
(1) vacuum treating the Al₂O₃ carrier for 10 mins to 30 mins at 100 °C to 150 °C, or directly heating for 1 hour to 10 hours at 100 °C to 150 °C, and then cooling to a room temperature;
(2) preparing a nickel salt solution having a nickel content of 0.05 g/mL to 0.2 g/mL;
(3) impregnating the nickel salt solution prepared by step (2) into the carrier of step (1) according to a ratio of 80 mL to 130 mL of nickel salt solution for 100g of the Al₂O₃ carrier, standing for 20 mins to 120 mins, then drying for 2 hours to 24 hours at a temperature raised to 100 °C to 150 °C, and calcining for 1 hour to 24 hours at 350 °C to 500 °C raised at 2 °C/min to 10 °C/min in an air or nitrogen atmosphere to obtain a NiO/Al₂O₃ precursor;
(4) preparing an ethanol-water solution of silicon precursor with a concentration of 0.0001 g/mL to 0.0015 g/mL by calculating SiO₂, and adjusting a pH value of the ethanol-water solution to 8.0 to 8.5 by ammonia water;
(5) suspending the NiO/Al₂O₃ precursor obtained by step (3) in the solution prepared by step (4) according to an amount of 1000 mL to 2000 mL of ethanol-water solution of silicon precursor for 100g of Al₂O₃ in the precursor, stirring and refluxing for 1 hour to 24 hours at a constant temperature of 20 °C to 60 °C, centrifugal washing three to five times respectively with absolute ethyl alcohol, 0.01M dilute nitric acid and deionized water after filtration, and drying the sample for 2 hours to 24 hours at 80 °C to 150 °C;
(6) calcining the sample obtained by step (5) for 1 hour to 24 hours at a constant temperature of 400 °C to 550 °C raised at 2 °C/min to 10 °C/min in an air or nitrogen atmosphere, and then conducting reduction for 1 hour to 24 hours at 350°C to 650°C under the condition of a gas space velocity (GSV) of hydrogen of 500/h to 2000/h to obtain a catalyst.

Regarding the preparation method of the Ni-Al₂O₃@SiO₂ catalyst with coated structure according to the invention, the nickel salt in the nickel salt water solution of step (2) is preferably one of a nickel nitrate, a nickel sulfate or a nickel chloride.

Regarding the preparation method of the Ni-Al₂O₃@SiO₂ catalyst with coated structure according to the invention, a drying time in step (3) is preferably 3 hours to 12 hours.

Regarding the preparation method of the Ni-Al₂O₃@SiO₂ catalyst with coated structure according to the invention, the silicon precursor in step (4) is preferably one of tetraethoxysilane (TEOS), or tetramethoxysilane (TMOS).

Regarding the preparation method of the Ni-Al₂O₃@SiO₂ catalyst with coated structure according to the invention, the silicon precursor in step (4) is further preferably TE-OS.

Regarding the preparation method of the Ni-Al₂O₃@SiO₂ catalyst with coated structure according to the invention, a molar ratio of ethanol to water in the ethanol-water solution of step (4) is preferably 4 ∼ 100:1.

Regarding the preparation method of the Ni-Al₂O₃@SiO₂ catalyst with coated structure according to the invention, a drying time in step (5) is preferably 3 hours to 12 hours.

In order to achieve the above object, the invention further provides an application of the Ni-Al₂O₃@SiO₂ catalyst with coated structure, wherein the catalyst is for hydrogenation to synthesize butane-1,4-diol using a Reppe process.

Regarding the application of the Ni-Al₂O₃@SiO₂ catalyst with coated structure according to the invention, comprising the step of:
the catalyst for hydrogenation to synthesize butane-1,4-diol using a Reppe process, and suitable for a fixed bed reactor, wherein a raw material is a water solution containing 25 wt% to 35 wt% butane-1,4-diol after prehydrogenation of a water solution of 1,4-butynediol at a low temperature and a low pressure, a way of feeding is up-in and down-out, and reaction conditions are a reaction temperature of 105 °C ∼ 150 °C, a hydrogen pressure of 10 MPa to 22 MPa, and a liquid hourly space velocity (LHSV) of 1.0/h ∼ 1.7/h.

Regarding the application of the Ni-Al₂O₃@SiO₂ catalyst with coated structure according to the invention, a carbonyl number of the water solution of butane-1,4-diol is preferably 7 mg(KOH)/g to 20 (KOH)/g.

Regarding the application of the Ni-Al₂O₃@SiO₂ catalyst with coated structure according to the invention, the raw material further preferably includes 1,4-butynediol, 1,4-butenediol, 4-hydroxybutyraldehyde, hemiacetal, 2-hydroxytetrahydrofuran and acetal, 2-(4'-hydroxybutoxy)-tetrahydrofuran.

Regarding the application of the Ni-Al₂O₃@SiO₂ catalyst with coated structure according to the invention, it may further comprise the step of:
the catalyst for hydrogenation to synthesize butane-1,4-diol using a Reppe process, and suitable for a fixed bed reactor, wherein a raw material is direct hydrogenation conversion of a water solution containing 25 wt% to 35 wt% 1,4-butynediol using an external cyclic hydrogenation process, a way of feeding is up-in and down-out, and reaction conditions are a reaction temperature of 105 °C ∼ 150 °C, a hydrogen pressure of 10 MPa to 22 MPa, a liquid hourly space velocity of 1.0/h ∼ 1.7/h, and a recycle ratio of 18 ∼ 22:1.

Regarding the application of the Ni-Al₂O₃@SiO₂ catalyst with coated structure according to the invention, an organic phase product of the material after hydrogenation contains butane-1,4-diol greater than or equal to 96 wt%, by-product butanol less than or equal to 1.2 wt%, and a total unsaturated chromogenic substance less than or equal to 0.06 wt%, the remaining is macromolecular polymer, and the carbonyl number of the material is 0.03 mg(KOH)/g to 0.05 mg(KOH)/g.

As compared to the prior arts, the invention has the following advantages:
1. The active component Ni is loaded onto the Al₂O₃ carrier using an impregnation method, Ni being distributed in tetrahedral and octahedral holes on the Al₂O₃ surface, which are used as nuclei to grow into microcrystalline particles. The particles have a high dispersity.
2. The SiO₂ layer is loaded in a grafting manner onto a surface of a Ni/Al₂O₃ catalyst, and since SiO₂ does not react with NiO, and interaction therebetween is extremely weak, SiO₂ only reacts with Al₂O₃ exposed in gaps of the NiO particles to produce corresponding Al-O-Si bonds. With extension of the grafting time, when the exposed Al₂O₃ surface is fully covered by a single layer of SiO₂, two, three, four...layers of SiO₂ are further grown on the surface of the exposed single layer of SiO₂.
3. Weak adsorption compositions remaining on the surface of the catalyst during grafting process, in particular, the SiO₂ layer weakly adsorbed on the NiO surface, is effectively removed by washing a sample after completion of grafting reaction with absolute ethyl alcohol, 0.01M dilute nitric acid and deionized water sequentially, which ensures effective exposure of the active component Ni particles.
4. During preparation of the traditional catalyst, due to strong interaction between NiO and Al₂O₃, reduction of NiO to the active metal Ni is often conducted in a high temperature, while high reduction temperature promotes migration and aggregation of the metal Ni, reduces the number of surface active sites, and also correspondingly reduces hydrogenation activity. In the invention, the SiO₂ layer is disposed between gaps of the NiO particles, and migration and aggregation of the metal Ni during the high temperature reduction can be effectively prevented by a confinement effect, so that the active metal nickel remains a high dispersity, and the catalyst exhibits a high hydrogenation activity.
5. The Al-O-Si structure formed between the SiO₂ layer and the Al₂O₃ carrier enables it to have a specific surface acidic property, which facilitates hydrolyzation of cyclic acetal, 2-(4'-hydroxybutoxy)-tetrahydrofuran to 4-hydroxybutyraldehyde, and then hydrogenation conversion to butane-1,4-diol under catalysis of adjacent high activity exposed hydrogenation center Ni. Synergistic effect of the acidic center and the hydrogenation active center in the catalyst achieves efficient conversion of cyclic acetal, 2-(4'-hydroxybutoxy)-tetrahydrofuran, and finally reaches objects of reducing the content of cyclic acetal, 2-(4'-hydroxybutoxy)-tetrahydrofuran, improving product quality of butane-1,4-diol, and reducing product chromaticity.
6. The SiO₂ layer is coated on the Al₂O₃ surface, which prevents attack of water to the Al₂O₃ carrier, inhibits dehydration structure of the Al₂O₃ carrier, effectively improves hydrothermal stability of the catalyst in a water containing system, and prolongs service life of the catalyst.

### BRIEF DESCRIPTION OF THE DRAWINGS

FIG. 1 is a structure diagram of the Ni-Al₂O₃@SiO₂ catalyst according to the invention.
FIG. 2 is an XRD diagram of the catalyst according to the invention.
FIG. 3 is a FT-IR diagram of the catalyst according to the invention.

### DETAILED DESCRIPTION OF THE INVENTION

Hereinafter examples of the invention are explained in detail: the examples are carried out on the premise of the technical solution of the invention with detailed embodiments and processes, but the extent of protection of the invention is not limited to the examples below. Generally, experimental method without indicating specific conditions in the examples follows conventional conditions.

The catalyst of the invention has a mass ratio of respective compositions Al₂O₃:SiO₂:Ni=100:0.1 ∼ 3: 4 ∼ 26, a specific surface area of 98 m²/g to 245 m²/g, and a pore volume of 0.25 cm³/g to 1.1 cm³/g. Ni particles are distributed on a surface of an Al₂O₃ carrier in an amorphous or highly dispersed state and have a grain size less than 8 nm, and a SiO₂ layer is filled among the Ni particles.

The above-mentioned Al₂O₃ has a specific surface area of 110 m²/g to 260 m²/g, and a pore volume of 0.5 cm³/g to 1.3 cm³/g.

A preparation method of the Ni-Al₂O₃@SiO₂ catalyst provided by the invention comprises steps of:
(1) vacuum treating the Al₂O₃ carrier for 10 mins to 30 mins at 100 °C to 150 °C, or directly heating for 1 hour to 10 hours at 100 °C to 150 °C, and then cooling to a room temperature;
(2) preparing a nickel salt solution having a nickel content of 0.05 g/mL to 0.2 g/mL;
(3) impregnating the nickel salt solution prepared by step (2) into the carrier of step (1) according to a ratio of 80 mL to 130 mL of nickel salt solution for 100g of the Al₂O₃ carrier, standing for 20 mins to 120 mins, then drying for 2 hours to 24 hours, preferably 3 hours to 12 hours, at a temperature raised to 100 °C to 150 °C, and calcining for 1 hour to 24 hours at 350 °C to 500 °C raised at 2 °C/min to 10 °C/min in an air or nitrogen atmosphere to obtain a NiO/Al₂O₃ precursor;
(4) preparing an ethanol-water solution of silicon precursor with a concentration of 0.0001 g/mL to 0.0015 g/mL by calculating SiO₂, and adjusting a pH value of the ethanol-water solution to 8.0 to 8.5 by ammonia water;
(5) suspending the NiO/Al₂O₃ precursor obtained by step (3) in the solution prepared by step (4) according to an amount of 1000 mL to 2000 mL of ethanol-water solution of silicon precursor for 100g of Al₂O₃ in the NiO/Al₂O₃ precursor, stirring and refluxing for 1 hour to 24 hours at a constant temperature of 20 °C to 60 °C, centrifugal washing three to five times respectively with absolute ethyl alcohol, 0.01M dilute nitric acid and deionized water after filtration, and drying the sample for 2 hours to 24 hours, preferably 3 hours to 12 hours, at 80 °C to 150 °C;
(6) calcining the sample obtained by step (5) for 1 hour to 24 hours at a constant temperature of 400 °C to 550 °C raised at 2 °C/min to 10 °C/min in an air or nitrogen atmosphere, and then conducting reduction for 1 hour to 24 hours at 350°C to 650°C under the condition of a gas space velocity of hydrogen of 500/h to 2000/h to obtain a catalyst.

The nickel salt in step (2) is one of a nickel nitrate, a nickel sulfate or a nickel chloride.

The silicon precursor in step (4) is one of tetraethoxysilane (TEOS), or tetramethoxysilane (TMOS), preferably TEOS, and a molar ratio of ethanol to water in the ethanol-water solution is 4 ∼ 100:1.

An application of the catalyst in the invention comprises step of:
The catalyst prepared in the invention is for hydrogenation to synthesize butane-1,4-diol using a Reppe process. (1) It is suitable for a fixed bed reactor, wherein a raw material is a water solution containing 25 wt% to 35 wt% butane-1,4-diol after prehydrogenation of a water solution of 1,4-butynediol at a low temperature and a low pressure (the solution has a carbonyl number of 7 mg(KOH)/g to 20 mg(KOH)/g, and contains impurities of 1,4-butynediol, 1,4-butenediol, 4-hydroxybutyraldehyde, hemiacetal and acetal, 2-(4'-hydroxybutoxy)-tetrahydrofuran, etc.), a way of feeding is up-in and down-out, and reaction conditions are a reaction temperature of 105 °C ∼ 150 °C, a hydrogen pressure of 10 MPa to 22 MPa, and a liquid hourly space velocity of 1.0/h ∼ 1.7/h. (2) On the fixed bed reactor, it also may be suitable for direct hydrogenation conversion of a raw material which is a water solution containing 25 wt% to 35 wt% 1,4-butynediol using an external cyclic hydrogenation process, a way of feeding is up-in and down-out, and reaction conditions are a reaction temperature of 105 °C ∼ 150 °C, a hydrogen pressure of 10 MPa to 22 MPa, a liquid hourly space velocity of 1.0/h ∼ 1.7/h, and a recycle ratio of 18 ∼ 22:1. An organic phase of the material after hydrogenation using the two processes contains butane-1,4-diol greater than or equal to 96 wt%, by-product butanol less than or equal to 1.2 wt%, and a total unsaturated chromogenic substance of 1,4-butynediol, 1,4-butenediol, 4-hydroxybutyraldehyde, hemiacetal and acetal, 2-(4'-hydroxybutoxy)-tetrahydrofuran less than or equal to 0.06 wt%, the remaining is macromolecular polymer, and the carbonyl number of the material is 0.03 mg(KOH)/g to 0.05 mg(KOH)/g. After rectification, it can be obtained that a product purity is 99.6 wt% to 99.8 wt%, the carbonyl number is 0.03 mg(KOH)/g to 0.05 mg(KOH)/g, and a chromaticity is less than or equal to 5APHA. Service life of the hydrogenation catalyst may reach to seventeen months.

### Example 1

(1) The Al₂O₃ carrier having a specific surface area of 110 m²/g, and a pore volume of 0.5 cm³/g was vacuum treated for 10 mins at 100 °C, and then cooled to a room temperature for use; a nickel nitrate solution having a nickel content of 0.05 g/mL was prepared; 80 mL of the prepared nickel salt solution was impregnated into 100g of the Al₂O₃ carrier, stood for 20 mins, dried for 2 hours at a temperature raised to 100 °C, and calcined for 1 hour at a constant temperature of 350 °C raised at 2 °C/min in an air atmosphere to obtain a NiO/Al₂O₃ precursor having a mass ratio of Al₂O₃:Ni to be 100:4.

The ethanol-water solution of silicon precursor with a concentration of 0.0001 g/mL by calculating SiO₂ was prepared from TEOS, a molar ratio of ethanol to water in the ethanol-water solution was 4:1, and a pH value of the ethanol-water solution was adjusted to be 8.0 by ammonia water. The prepared NiO/Al₂O₃ precursor was suspended in 1000 mL of ethanol-water solution of silicon precursor, stirred and refluxed for 1 h at a constant temperature of 20 °C, centrifugal washed three times respectively with absolute ethyl alcohol, 0.01M dilute nitric acid and deionized water after filtration, and the sample was dried for 2 hours at 80 °C. The obtained sample was placed in a muffle furnace to calcine for 1 hour at a constant temperature of 400 °C raised at 2 °C/min in an air atmosphere, and then reduction was conducted for 1 hour at 350°C and a gas space velocity of hydrogen of 500/h to obtain a Ni-Al₂O₃@SiO₂ catalyst with a quality composition of Al₂O₃:SiO₂:Ni=100:0.1:4. The catalyst had a specific surface area of 105 m²/g, and a pore volume of 0.45 cm³/g, Ni particles were being in an amorphous state, the SiO₂ layer filled among the Ni particles, and Al-O-Si bonds were formed between the SiO₂ layer and Al₂O₃. The catalyst was numbered as 1#.

### Example 2

(1) The Al₂O₃ carrier having a specific surface area of 110 m²/g, and a pore volume of 0.5 cm³/g was vacuum treated for 30 mins at 150 °C, and then cooled to a room temperature for use; a nickel salt solution of nickel sulfate having a nickel content of 0.2 g/mL was prepared; 130 mL of the prepared nickel salt solution was impregnated into 100g of the Al₂O₃ carrier, stood for 120 mins, dried for 24 hours at a temperature raised to 150 °C, and calcined for 24 hours at a constant temperature of 500°C raised at 10°C/min in a nitrogen atmosphere to obtain a NiO/Al₂O₃ precursor having a mass ratio of Al₂O₃:Ni to be 100:26.

The ethanol-water solution of silicon precursor with a concentration of 0.0015 g/mL by calculating SiO₂ was prepared from TMOS, a molar ratio of ethanol to water in the ethanol-water solution was 100:1, and a pH value of the ethanol-water solution was adjusted to be 8.5 by ammonia water. The NiO/Al₂O₃ precursor prepared with the mass ratio of Al₂O₃:Ni to be 100:26 was suspended in 2000 mL of ethanol-water solution of silicon precursor, stirred and refluxed for 24 hours at a constant temperature of 60°C, centrifugal washed five times respectively with absolute ethyl alcohol, 0.01M dilute nitric acid and deionized water after filtration, and the sample was dried for 24 hours at 150 °C. The obtained sample was placed in a muffle furnace to calcine for 24 hours at a constant temperature of 550 °C raised at 10 °C/min in a nitrogen atmosphere, and then reduction was conducted for 24 hours at 650°C and a gas space velocity of hydrogen of 2000/h to obtain a Ni-Al₂O₃@SiO₂ catalyst having a quality composition of Al₂O₃:SiO₂:Ni=100:3:26. The catalyst had a specific surface area of 98 m²/g, and a pore volume of 0.25 cm³/g, Ni particles were distributed on a surface of the Al₂O₃ carrier in a highly dispersed state and had a grain size of 8 nm, the SiO₂ layer filled among the Ni particles, and Al-O-Si bonds were formed between the SiO₂ layer and Al₂O₃. The catalyst was numbered as 2#.

### Example 3

(1) The Al₂O₃ carrier having a specific surface area of 260 m²/g, and a pore volume of 1.3 cm³/g was heated for 1 hour at 100 °C in an air-circulating oven, and then cooled to a room temperature for use; a nickel salt solution of nickel chloride having a nickel content of 0.05 g/mL was prepared; 80 mL of the prepared nickel salt solution was impregnated into 100g of the Al₂O₃ carrier, stood for 80 mins, dried for 3 hours at a temperature raised to 120 °C, and calcined for 18 hours at a constant temperature of 350°C raised at 8°C/min in an air atmosphere to obtain a NiO/Al₂O₃ precursor having a mass ratio of Al₂O₃:Ni to be 100:4.

The ethanol-water solution of silicon precursor with a concentration of 0.0001 g/mL by calculating SiO₂ was prepared from TMOS, a molar ratio of ethanol to water in the ethanol-water solution was 10:1, and a pH value of the ethanol-water solution was adjusted to be 8.2 by ammonia water. The NiO/Al₂O₃ precursor prepared with the mass ratio of Al₂O₃:Ni to be 100:4 was suspended in 1000 mL of ethanol-water solution of silicon precursor, stirred and refluxed for 12 hours at a constant temperature of 40 °C, centrifugal washed four times respectively with absolute ethyl alcohol, 0.01M dilute nitric acid and deionized water after filtration, and the sample was dried for 3 hours at 150 °C. The obtained sample was placed in a muffle furnace to calcine for 12 hours at a constant temperature of 500 °C raised at 8°C/min in a nitrogen atmosphere, and then reduction was conducted for 10 hours at 550°C and a gas space velocity of hydrogen of 1000/h to obtain a Ni-Al₂O₃@SiO₂ catalyst having a quality composition of Al₂O₃:SiO₂:Ni=100:0.1:4. The catalyst had a specific surface area of 245 m²/g, and a pore volume of 1.10 cm³/g, Ni particles were distributed on a surface of the Al₂O₃ carrier in an amorphous state, the SiO₂ layer filled among the Ni particles, and Al-O-Si bonds were formed between the SiO₂ layer and Al₂O₃. The catalyst was numbered as 3#.

### Example 4

(1) The Al₂O₃ carrier having a specific surface area of 160 m²/g, and a pore volume of 0.75 cm³/g was heated for 10 hours at 150 °C in an air-circulating oven, and then cooled to a room temperature for use; a nickel salt solution of nickel nitrate having a nickel content of 0.10 g/mL was prepared; 95 mL of the prepared nickel salt solution was impregnated into 100g of the Al₂O₃ carrier, stood for 80 mins, dried for 12 hours at a temperature raised to 120 °C, and calcined for 5 hours at a constant temperature of 350°C raised at 2°C/min in an air atmosphere to obtain a NiO/Al₂O₃ precursor having a mass ratio of Al₂O₃:Ni to be 100:9.5.

The ethanol-water solution of silicon precursor with a concentration of 0.0008 g/mL by calculating SiO₂ was prepared from TEOS, a molar ratio of ethanol to water in the ethanol-water solution was 20:1, and a pH value of the ethanol-water solution was adjusted to be 8.4 by ammonia water. The NiO/Al₂O₃ precursor prepared with the mass ratio of Al₂O₃:Ni to be 100:9.5 was suspended in 1300 mL of ethanol-water solution of silicon precursor, stirred and refluxed for 24 hours at a constant temperature of 20°C, centrifugal washed five times respectively with absolute ethyl alcohol, 0.01M dilute nitric acid and deionized water after filtration, and the sample was dried for 12 hours at 100 °C. The obtained sample was placed in a muffle furnace to calcine for 1 hour at a constant temperature of 400°C raised at 10°C/min in a nitrogen atmosphere, and then reduction was conducted for 24 hours at 450°C and a gas space velocity of hydrogen of 1500/h to obtain a Ni-Al₂O₃@ SiO₂ catalyst having a quality composition of Al₂O₃:SiO₂:Ni=100:1.04:9.5. The catalyst had a specific surface area of 145 m²/g, and a pore volume of 0.61 cm³/g, Ni particles were distributed on a surface of the Al₂O₃ carrier in a highly dispersed state and had a grain size of 6.3 nm, the SiO₂ layer filled among the Ni particles, and Al-O-Si bonds were formed between the SiO₂ layer and Al₂O₃. The catalyst was numbered as 4#.

### Example 5

The Al₂O₃ carrier having a specific surface area of 210 m²/g, and a pore volume of 1.0 cm³/g was heated for 5 hours at 130 °C in an air-circulating oven, and then cooled to a room temperature for use; a water solution of nickel nitrate having a nickel content of 0.15 g/mL was prepared; 110 mL of the prepared nickel salt solution was impregnated into 100g of the Al₂O₃ carrier, stood for 20 mins, dried for 3 hours at a temperature raised to 100 °C, and calcined for 1 hour at a constant temperature of 350 °C raised at 10 °C/min in an air atmosphere to obtain a NiO/Al₂O₃ precursor having a mass ratio of Al₂O₃:Ni to be 100:16.5.

The ethanol-water solution of silicon precursor with a concentration of 0.0012 g/mL by calculating SiO₂ was prepared from TMOS, a molar ratio of ethanol to water in the ethanol-water solution was 4:1, and a pH value of the ethanol-water solution was adjusted to be 8.0 by ammonia water. The NiO/Al₂O₃ precursor prepared with the mass ratio of Al₂O₃:Ni to be 100:16.5 was suspended in 1700 mL of ethanol-water solution of silicon precursor, stirred and refluxed for 24 hours at a constant temperature of 20°C, centrifugal washed three times respectively with absolute ethyl alcohol, 0.01M dilute nitric acid and deionized water after filtration, and the sample was dried for 3 hours at 80 °C. The obtained sample was placed in a muffle furnace to calcine for 1 hour at a constant temperature of 400°C raised at 2°C/min in an air atmosphere, and then reduction was conducted for 1 hour at 350°C and a gas space velocity of hydrogen of 750/h to obtain a Ni-Al₂O₃@SiO₂ catalyst having a quality composition of Al₂O₃:SiO₂:Ni=100:2.04:16.5. The catalyst had a specific surface area of 183 m²/g, and a pore volume of 0.85 cm³/g, Ni particles were distributed on a surface of the Al₂O₃ carrier in a highly dispersed state and had a grain size of 7.5 nm, the SiO₂ layer filled among the Ni particles, and Al-O-Si bonds were formed between the SiO₂ layer and Al₂O₃. The catalyst was numbered as 5#.

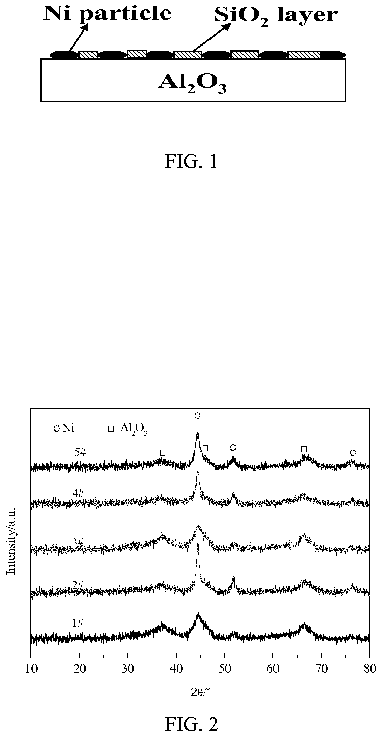

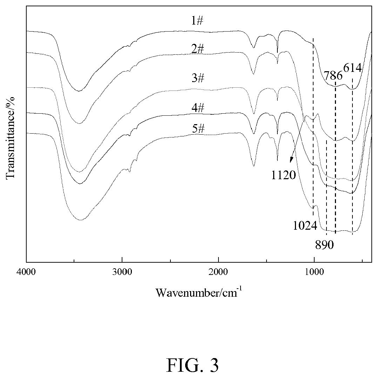

Catalyst structures of the catalysts 1#-5# are characterized by XRD, IR and XPS techniques. XRD diagrams of the reduced catalysts are illustrated in FIG. 2. All catalysts exhibit dispersed characteristic diffraction peaks of γ-Al₂O₃ at 2θ = 37°, 46° and 66.5°, and exhibit characteristic peaks of the metal Ni at 2θ = 44. 5°, 51. 7° and 76. 4°. Ni grain sizes are calculated by the formula Scherrer using a full width at half maximum of the Ni (200) crystal plane diffraction peak at 2θ = 51. 7°, which are listed in Table 1. As can be seen, Ni particles are highly dispersed, and the grain sizes are less than 8 nm. FT-IR diagrams of respective catalysts are illustrated in FIG. 3. As can be seen, except observing a four-coordinated Al-O vibration absorption peak at 890 cm⁻¹, and six-coordinated Al-O vibration absorption peaks at 786cm⁻¹ and 614cm⁻¹, all samples appear new infrared absorption peaks at 1024 cm⁻¹, which belong to vibration absorption peaks of the Si-O-Al bonds. This result shows that the loaded SiO₂ is bonded with Al₂O₃ to form new Si-O-Al bonds. In the sample 3#, stretching vibration of Si-O-Si at 1120 cm⁻¹ is further observed, which indicates that volume phase SiO₂ is formed in the high Si content. After further XPS analysis on the catalysts, atomic ratios of Ni/(Al+Si) on surfaces of the catalysts 1#-5# are calculated in accordance with XPS data. In order to make comparison, atomic ratios of Ni/Al in the Ni/Al₂O₃ catalyst when Si is not loaded are also calculated, and results are listed in Table 1. As can be seen, the atomic ratios of Ni/Al before loading the SiO₂ layer onto the catalyst are in close proximity to the atomic ratios of Ni/(Al+Si) after loading SiO₂, and it is further determined that the loaded SiO₂ layer is selectively deposited on the Al₂O₃ surface to form the Si-O-Al bonds and form the coating structure shown in FIG. 1.

**Table 1 Ni Grain Size and Ni Surface Concentration**

| No. | Average Ni Grain Size | Ratio of Ni/(Si+Al) | Ratio of Ni/Al of the Sample Before Loading SiO₂ |
|---|---|---|---|
| 1# | -- | 0.041 | 0.042 |
| 2# | 8 | 0.70 | 0.71 |
| 3# | -- | 0.043 | 0.043 |
| 4# | 6.3 | 0.18 | 0.19 |
| 5# | 7.5 | 0.51 | 0.51 |

### Example 6

The catalysts 1#-5# and the available commercial catalyst (manufacturer: Degussa°Ctolyst™ 1003) (numbered as 6#) were selected. (1) On a fixed bed reactor, a water solution containing 25 wt% to 35 wt% butane-1,4-diol after prehydrogenation of a water solution of 1,4-butynediol at a low temperature and a low pressure (the solution had a carbonyl number of 7 mg(KOH)/g to 20 mg(KOH)/g, and contained impurities of 1,4-butynediol, 1,4-butenediol, 4-hydroxybutyraldehyde, hemiacetal and acetal, 2-(4'-hydroxybutoxy)-tetrahydrofuran, etc.) was used as a raw material, a way of feeding was up-in and down-out, and reaction conditions were a reaction temperature of 105 °C ∼ 150 °C, a hydrogen pressure of 10 MPa to 22 MPa, and a liquid hourly space velocity of 1.0/h ∼ 1.7/h. Specific reaction conditions and results are shown in Table 2.

(2) On the fixed bed reactor, a water solution containing 25 wt% to 35 wt% 1,4-butynediol was used as a raw material using an external cyclic hydrogenation process, a way of feeding was up-in and down-out, and reaction conditions were a reaction temperature of 105 °C ∼ 150 °C, a hydrogen pressure of 10 MPa to 22 MPa, a liquid hourly space velocity of 1.0/h ∼ 1.7/h, and a recycle ratio of 18 ∼ 22:1. Specific reaction conditions and results are shown in Table 3.

An organic phase of the material after hydrogenation using the two processes contains butane-1,4-diol greater than or equal to 96 wt%, by-product butanol less than or equal to 1.2 wt%, and a total unsaturated chromogenic substance of 1,4-butynediol, 1,4-butenediol, 4-hydroxybutyraldehyde, hemiacetal and acetal, 2-(4'-hydroxybutoxy)-tetra-hydrofuran less than or equal to 0.06 wt%, the remaining is macromolecular polymer, and the carbonyl number of the material is 0.03 mg(KOH)/g to 0.05 mg(KOH)/g. After rectification, it can be obtained that a product purity is 99.6 wt% to 99.8 wt%, the carbonyl number is 0.03 mg(KOH)/g to 0.05 mg(KOH)/g, and a chromaticity is less than or equal to 5APHA. Service life of the hydrogenation catalyst may reach to fifteen to eighteen months.

**Table 2**

| No. | Raw Material Index | | Reaction Conditions | | | Compositions of Organic Phase of Hydrogenated Material | | | | Carbonyl number/mg(KOH)/g |
|---|---|---|---|---|---|---|---|---|---|---|
| | butane-1,4-diol/wt% | Carbonyl number/mg(KOH)/g | Temperature/°C | Pressure/MPa | A liquid hourly space velocity/h | butane-1,4-diol /wt% | Butanol/wt% | Unsaturated Chromogenic Substance /wt% | Other/wt% | |
| 1# | 27 | 13.8 | 135 | 13 | 1.2 | 97 | 0.7 | 0.06 | 2.24 | 0.03 |
| 2# | 26 | 17.2 | 110 | 22 | 1.3 | 97 | 0.8 | 0.06 | 2.14 | 0.04 |
| 3# | 30 | 7 | 120 | 10 | 1.7 | 98 | 0.6 | 0.05 | 1.35 | 0.03 |
| 4# | 25 | 20 | 105 | 15 | 1.0 | 97.3 | 1.2 | 0.06 | 1.44 | 0.04 |
| 5# | 28 | 10.5 | 150 | 17 | 1.5 | 96.17 | 1.2 | 0.03 | 2.6 | 0.05 |
| 6# | 25 | 20 | 150 | 20 | 1.0 | 90.2 | 6.3 | 0.7 | 2.8 | 0.4 |

**Table 3**

| No. | Raw Material Index | Reaction Conditions | | | | Compositions of Organic Phase of Hydrogenated Material | | | | Number of Carbonyl Groups /mg(KOH)/g |
|---|---|---|---|---|---|---|---|---|---|---|
| | 1,4-butynediol/wt% | Temperature/°C | Pressure/MPa | A liquid hourly space velocity/h | Recycle Ratio | butane-1,4-diol/wt% | Butanol/wt% | Unsaturated Chromogenic Substance/wt% | Other/wt% | |
| 1# | 28 | 135 | 13 | 1.2 | 19:1 | 98.11 | 0.6 | 0.04 | 1.25 | 0.03 |
| 2# | 32 | 110 | 22 | 1.3 | 18:1 | 96.16 | 1.2 | 0.05 | 2.59 | 0.03 |
| 3# | 30 | 150 | 17 | 1.5 | 22:1 | 97 | 0.8 | 0.06 | 2.14 | 0.05 |
| 4# | 35 | 120 | 10 | 1.7 | 20:1 | 97.41 | 1.0 | 0.05 | 1.54 | 0.04 |
| 5# | 25 | 105 | 15 | 1.0 | 18:1 | 96.85 | 0.7 | 0.03 | 2.42 | 0.05 |
| 6# | 25 | 150 | 20 | 1.0 | 18:1 | 90.3 | 7.3 | 1.2 | 1.2 | 0.6 |

### Industrial Applicability

The catalyst of the invention is for hydrogenation to synthesize butane-1,4-diol using a Reppe process. The organic phase of the material after hydrogenation contains butane-1,4-diol greater than or equal to 96 wt%, by-product butanol less than or equal to 1.2 wt%, and a total unsaturated chromogenic substance of 1,4-butynediol, 1,4-butenediol, 4-hydroxybutyraldehyde, hemiacetal and acetal, 2-(4'-hydroxybutoxy)-tetrahydrofuran less than or equal to 0.06 wt%, the remaining is macromolecular polymer, and the carbonyl number of the material is 0.03 mg(KOH)/g to 0.05 mg(KOH)/g. After rectification, it can be obtained that a product purity is 99.6 wt% to 99.8 wt%, the carbonyl number is 0.03 mg(KOH)/g to 0.05 mg(KOH)/g, and a chromaticity is less than or equal to 5APHA. Service life of the hydrogenation catalyst may reach to seventeen months.

Of course, the invention also may have many other examples, and those persons skilled in the art may make various corresponding modifications and variations according to the invention without departing from spirit and substance of the invention, but these corresponding modifications and variations shall belong to the extent of protection of the claims in the invention.

## Claims

1. A Ni-Al₂O₃@SiO₂ catalyst with coated structure, **characterized in that** Ni particles are distributed on a surface of an Al₂O₃ carrier in an amorphous or highly dispersed state and have a grain size less than or equal to 8 nm, and a SiO₂ layer is filled among the Ni particles.

2. The Ni-Al₂O₃@SiO₂ catalyst with coated structure according to claim 1, wherein the catalyst has a mass ratio of respective compositions Al₂O₃:SiO₂:Ni=100: 0.1 ∼ 3: 4 ∼ 26, a specific surface area of 98 m²/g ∼ 245 m²/g, and a pore volume of 0.25 cm³/g ∼ 1.1 cm³/g.

3. The Ni-Al₂O₃@SiO₂ catalyst with coated structure according to claim 2, wherein the Al₂O₃ carrier has a specific surface area of 110 m²/g ∼ 260 m²/g, and a pore volume of 0.5 cm³/g ∼ 1.3 cm³/g.

4. A preparation method of the Ni-Al₂O₃@SiO₂ catalyst with coated structure according to claim 1 or 2 or 3, comprising the steps of:
impregnation step: loading the active components Ni onto the Al₂O₃ carrier using an impregnation method, Ni being distributed in tetrahedral and octahedral holes on the Al₂O₃ surface, and growing into microcrystalline particles by using the tetrahedral and octahedral holes as nuclei;
grafting step: loading the SiO₂ layer in a grafting manner onto a surface of a Ni/Al₂O₃ catalyst obtained in the impregnation step, SiO₂ reacting with Al₂O₃ exposed in gaps of the Ni particles to produce corresponding Al-O-Si bonds; and
washing step: removing weak adsorption compositions remaining on the surface of the catalyst during grafting process by washing the sample after completion of grafting reaction.

5. The preparation method of the Ni-Al₂O₃@SiO₂ catalyst with coated structure according to claim 1 or 2 or 3, comprising the steps of:
(1) cooling the Al₂O₃ carrier to a room temperature for use after treatment at 100 °C to 150 °C;
(2) preparing a nickel salt water solution having a nickel content of 0.05 g/mL to 0.2 g/mL;
(3) impregnating the nickel salt water solution prepared by step (2) into the carrier of step (1) according to a ratio of 80 mL to 130 mL of nickel salt solution for 100g of the Al₂O₃ carrier, standing, drying and calcining to obtain a NiO/Al₂O₃ precursor;
(4) preparing an ethanol-water solution of silicon precursor with a concentration of 0.0001 g/mL to 0.0015 g/mL by calculating SiO₂, and adjusting a pH value of the ethanol-water solution to 8.0 to 8.5 by ammonia water;
(5) suspending the NiO/Al₂O₃ precursor obtained by step (3) in the solution prepared by step (4) according to an amount of 1000 mL to 2000 mL of ethanol-water solution of silicon precursor for 100g of Al₂O₃ in the precursor, stirring and refluxing at a constant temperature, centrifugal washing respectively with absolute ethyl alcohol, dilute nitric acid and deionized water after filtration, and drying;
(6) calcining the sample obtained by step (5), then conducting reduction under the condition of hydrogen to obtain a catalyst.

6. The preparation method of the Ni-Al₂O₃@SiO₂ catalyst with coated structure according to claim 2 or 3, comprising the steps of:
(1) vacuum treating the Al₂O₃ carrier for 10 mins to 30 mins at 100 °C to 150 °C, or directly heating for 1 hour to 10 hours at 100 °C to 150 °C, and then cooling to a room temperature;
(2) preparing a nickel salt solution having a nickel content of 0.05 g/mL to 0.2 g/mL;
(3) impregnating the nickel salt solution prepared by step (2) into the carrier of step (1) according to a ratio of 80 mL to 130 mL of nickel salt solution for 100g of the Al₂O₃ carrier, standing for 20 mins to 120 mins, then drying for 2 hours to 24 hours at a temperature raised to 100 °C to 150 °C, and calcining for 1 hour to 24 hours at 350 °C to 500 °C raised at 2 °C/min to 10 °C/min in an air or nitrogen atmosphere to obtain a NiO/Al₂O₃ precursor;
(4) preparing an ethanol-water solution of silicon precursor with a concentration of 0.0001 g/mL to 0.0015 g/mL by calculating SiO₂, and adjusting a pH value of the ethanol-water solution to 8.0 to 8.5 by ammonia water;
(5) suspending the NiO/Al₂O₃ precursor obtained by step (3) in the solution prepared by step (4) according to an amount of 1000 mL to 2000 mL of ethanol-water solution of silicon precursor for 100g of Al₂O₃ in the precursor, stirring and refluxing for 1 hour to 24 hours at a constant temperature of 20 °C to 60 °C, centrifugal washing three to five times respectively with absolute ethyl alcohol, 0.01M dilute nitric acid and deionized water after filtration, and drying the sample for 2 hours to 24 hours at 80 °C to 150 °C;
(6) calcining the sample obtained by step (5) for 1 hour to 24 hours at a constant temperature of 400 °C to 550 °C raised at 2 °C/min to 10 °C/min in an air or nitrogen atmosphere, and then conducting reduction for 1 hour to 24 hours at 350°C to 650°C under the condition of a liquid hourly space velocity of hydrogen of 500/h to 2000/h to obtain a catalyst.

7. The preparation method of the Ni-Al₂O₃@SiO₂ catalyst with coated structure according to claim 6, wherein the nickel salt in the nickel salt solution of step (2) is one of a nickel nitrate, a nickel sulfate or a nickel chloride.

8. The preparation method of the Ni-Al₂O₃@SiO₂ catalyst with coated structure according to claim 6, wherein a drying time in step (3) is 3 hours to 12 hours.

9. The preparation method of the Ni-Al₂O₃@SiO₂ catalyst with coated structure according to claim 6, wherein the silicon precursor in step (4) is one of tetraethoxysilane (TE-OS), or tetramethoxysilane (TMOS).

10. The preparation method of the Ni-Al₂O₃@SiO₂ catalyst with coated structure according to claim 6, wherein the silicon precursor in step (4) is TEOS.

11. The preparation method of the Ni-Al₂O₃@SiO₂ catalyst with coated structure according to claim 6, wherein a molar ratio of ethanol to water in the ethanol-water solution of step (4) is 4 ∼ 100:1.

12. The preparation method of the Ni-Al₂O₃@SiO₂ catalyst with coated structure according to claim 6, wherein a drying time in step (5) is 3 hours to 12 hours.

13. An application of the Ni-Al₂O₃@SiO₂ catalyst with coated structure according to claim 1 or 2 or 3, wherein the catalyst is for hydrogenation to synthesize butane-1,4-diol using a Reppe process.

14. The application of the Ni-Al₂O₃@SiO₂ catalyst with coated structure according to claim 13, comprising the step of:
the catalyst for hydrogenation to synthesize butane-1,4-diol using a Reppe process, and suitable for a fixed bed reactor, wherein a raw material is a water solution containing 25 wt% to 35 wt% butane-1,4-diol after prehydrogenation of a water solution of 1,4-butynediol at a low temperature and a low pressure, a way of feeding is up-in and down-out, and reaction conditions are a reaction temperature of 105 °C ∼ 150 °C, a hydrogen pressure of 10 MPa to 22 MPa, and a liquid hourly space velocity of 1.0/h ∼ 1.7/h.

15. The application of the Ni-Al₂O₃@SiO₂ catalyst with coated structure according to claim 14, wherein a carbonyl number of the water solution of butane-1,4-diol is 7 mg(KOH)/g to 20 (KOH)/g.

16. The application of the Ni-Al₂O₃@SiO₂ catalyst with coated structure according to claim 14 or 15, wherein the raw material further includes 1,4-butynediol, 1,4-butenediol, 4-hydroxybutyraldehyde, hemiacetal, 2-hydroxytetrahydrofuran and acetal, 2-(4'-hydroxy-butoxy)-tetrahydrofuran.

17. The application of the Ni-Al₂O₃@SiO₂ catalyst with coated structure according to claim 13, comprising the step of:
the catalyst for hydrogenation to synthesize butane-1,4-diol using a Reppe process, and suitable for a fixed bed reactor, wherein a raw material is direct hydrogenation conversion of a water solution containing 25 wt% to 35 wt% 1,4-butynediol using an external cyclic hydrogenation process, a way of feeding is up-in and down-out, and reaction conditions are a reaction temperature of 105 °C ∼150 °C, a hydrogen pressure of 10 MPa to 22 MPa, a liquid hourly space velocity of 1.0/h ∼ 1.7/h, and a recycle ratio of 18 ∼ 22:1.

18. The application of the Ni-Al₂O₃@SiO₂ catalyst with coated structure according to claim 14, 15 or 17, wherein an organic phase product of the material after hydrogenation contains butane-1,4-diol greater than or equal to 96 wt%, by-product butanol less than or equal to 1.2 wt%, and a total unsaturated chromogenic substance less than or equal to 0.06 wt%, the remaining is macromolecular polymer, and the carbonyl number of the material is 0.03 mg(KOH)/g to 0.05 mg(KOH)/g.
